# EUROPEAN PATENT SPECIFICATION

(11) **EP 0 558 213 B1**
(45) Date of publication and mention of the grant of the patent: **26.06.1996**
(21) Application number: 93301059.7
(22) Date of filing: 15.02.1993
(51) Int. Cl.: C12P 19/12, C12P 19/14

(54) **Process for preparing neotrehalose, and its uses**
Verfahren zur Herstellung von Neotrehalose und deren Verwendungen
Procédé de préparation de néotréhalose et ses utilisations

(30) Priority: 25.02.1992 JP 93936/92
(43) Date of publication of application: 01.09.1993
(73) Proprietor: KABUSHIKI KAISHA HAYASHIBARA SEIBUTSU KAGAKU KENKYUJO, Okayama-shi Okayama (JP)
(72) Inventor: Shibuya, Takashi, Souja-shi, Okayama (JP); Chaen, Hiroto, Okayama-shi, Okayama (JP); Sakai, Shuzo, Seto-cho, Akaiwa-gun, Okayama (JP); Miyake, Toshio, Okayama-shi, Okayama (JP)
(74) Representative: Daniels, Jeffrey Nicholas

(56) References cited:
- PATENT ABSTRACTS OF JAPAN, unexamined applications, C field, vol. 13, no. 3, 06 January 1989, The Patent Office Japanese Govt.; p. 150 C 557
- PATENT ABSTRACTS OF JAPAN, unexamined applications, C field, vol. 13, no. 42, 30 January 1989, The Patent Office Japanese Govt.; p. 54 C 564

## Description

The present invention relates to a process for preparing neotrehalose, and its uses; more particularly, to a process which comprises allowing α-amylase to act on an amylaceous substance to form neotrehalose, and recovering said neotrehalose, and to a composition such as food products and pharmaceuticals which contain said neotrehalose.

Neotrehalose has been known for long as a disaccharide as shown by the formula O-α-D-glucopyranosyl β-D-glucopyranoside or O-β-D-glucopyranosyl α-D-glucopyranoside. The saccharide has a rich sweetness, readily water-solubility, non-reducing activity and satisfiable stability, and because of these it has been expected to use in products such as beverages, processed foods, tobaccos and cigarettes.

As described by W. H. Haworth in Journal of Chemical Society, pp.2847-2850 (1931) and by V. E. S. Sharp in ibid, pp.285-288 (1951), preparations of neotrehalose via a chemical synthesis have been known. There still remains, however, many problems which should be overcome before the actual use of neotrehalose in view of the safeness and yield, i.e. an unfavorably-low yield, because neotrehalose has been chemically synthesized.

Preparations of neotrehalose from natural sources having a relatively-high safeness or other preparations wherein a biochemical synthesis is used have been proposed. For example, Matsuda reported in Nippon Nogeikagaku Kaishi, Vol.30, pp.119-123 (1959) that a small amount of neotrehalose is present in a kojic extract together with isomaltose and kojibiose. According to this publication, neotrehalose is prepared by a method comprising subjecting a non-fermentable saccharide contained in a kojic extract to column chromatography wherein a carbon is used, recovering a disaccharide-rich fraction, treating the fraction with phenylhydrazine, separating the resultant sediment with a filter, acetylating saccharides in the filtrate to obtain a crystalline disaccharide octaacetate, and deacetylating the resultant crystal. Z. L. Nikolov et al. in Biotechnology and Bioengineering, Vol.34, pp.694-704 (1989) revealed that neotrehalose is formed from glucose in a yield of 0.43 w/w % (hereinafter the symbol "w/w %" is abbreviated as "%", if specified otherwise) when subjected to the condensing action of glucoamylase.

Kobayashi et al. discloses in Japanese Patent Laid-Open No.216,492/88 a preparation of neotrehalose which comprises allowing a cyclodextrin-forming enzyme to act on an amylaceous substance to form neotrehalose together with centose. The publication describes that high-performance liquid chromatography (HPLC) analysis revealed that a reaction mixture, which had been prepared by allowing a cyclodextrin-forming enzyme to act on an amylaceous substance, contained 18.6% neotrehalose and 20.0% centose, d.s.b.; and that paper chromatography analysis revealed that the resultant neotrehalose-rich fraction contained about 20% nigerose, kojibiose and isomaltose, d.s.b. They also proposed a preparation of neotrehalose from the reaction mixture, said preparation comprising deactivating the remaining enzyme in the reaction mixture, simultaneously adding glucoamylase and yeast to the mixture in order to assimilate and remove the formed glucose, removing the yeast, adding sodium hydroxide to the mixture, autoclaving the resultant mixture, neutralizing the autoclaved mixture, and subjecting the neutralized mixture to column chromatography and/or sedimentation with an organic solvent. These two preparations are complicated in the purification steps, and the yields of neotrehalose are relatively low so that they have not yet been industrially practiced. The present inventors disclosed in Japanese Patent Application No.307,054/90 a process for preparing neotrehalose, and its uses, said process comprising allowing ß-galactosidase to act on lactoneotrehalose (O-β-D-galactopyranosyl-(1→4)-O-β-D-glucopyranosyl α-D-glucopyranoside) in order to form neotrehalose, and recovering said neotrehalose. The process enables a preparation of neotrehalose in a relatively-high yield against the material lactoneotrehalose, while some problems in the preparation of the material were remained.

For the actual use of neotrehalose, it has been a great demand to establish a preparation of neotrehalose having a satisfiable safeness and being readily prepared in a relatively-high yield, and its uses.

The present inventors have studied to establish a preparation of neotrehalose, and its uses; more particularly, we studied on enzymes which hydrolyze amylaceous substances.

As a result, we found that neotrehalose is formed by allowing α-amylase (EC 3.2.1.1) to act on an amylaceous substance, and established a composition which contains said neotrehalose. Thus, we accomplished this invention.

The invention will now be described in further detail by way of example only, with reference to the accompanying drawings, in which:
FIG.1 shows a chromatogram of an enzymatic reaction mixture on HPLC;
FIG.2 shows a chromatogram of an enzymatic reaction mixture on gas chromatography.

The present invention relates to a process for preparing neotrehalose, arid its uses; more particularly, to a process which comprises allowing α-amylase to act on an amylaceous substance to form neotrehalose, and recovering said neotrehalose, and to a composition such as food products and pharmaceuticals which contain said neotrehalose.

The amylaceous substances usable in the invention are starch and its partial hydrolysates, or, usually those having a dextrose equivalent (DE) of about 70 or lower. Examples of such amylaceous substances are gelatinized starch, liquefied starch, solubilized starch, partial starch hydrolysates, and amylaceous saccharide-transferred products. Amylaceous substances from subterranean stem such as potato, tapioca and sweet potato; and those derived from terrestrial stem such as corn, wheat and rice are suitably used in the invention as a material starch.

The starch hydrolysates suitably used in the invention are high-molecular weight products such as amylose and dextrins, as well as one or more maltooligosaccharides such as maltose, maltotriose, maltotetraose, maltopentaose, maltohexaose, maltoheptaose and maltooctaose.

The α-amylases usable in the invention are those which can form neotrehalose when acted on amylaceous substances. For example, those derived from microorganisms of the genera Bacillus and Aspergillus can be suitably used. We found that among these α-amylases a saccharogenic α-amylase is suitably used to form neotrehalose, and that such a saccharogenic α-amylase is of microorganisms of the species Bacillus subtilis because it forms neotrehalose in a relatively-high yield, as well as having a great advantage in an industrial-scale preparation.

As described in Denpun Handbook (Starch Handbook), pp.106-108, edited by Jiro Nikuni, published by Asakura Publishing Company, Tokyo, Japan, (1967), the above-mentioned saccharogenic α-amylase is an α-amylase which has an extremely-high saccharification power as compared with its dextrinogenic power.

Any enzymatic reaction can be used in the invention as long as it forms neotrehalose. For example, in case of using a saccharogenic α-amylase derived from microorganisms of the species Bacillus subtilis, neotrehalose is formed by subjecting an aqueous solution containing an amylaceous substance such as partial starch hydrolysates to the action of 1-1,000 units/g amylaceous substance, based on the weight of the dry solid (d.s.b.), of the saccharogenic α-amylase. In this case, the enzymatic reaction is effected under the conditions selected from a concentration in the range of 1-80%, d.s.b., preferably, 20-70%, d.s.b., of amylaceous substances; a temperature in the range of 20-80°C; a pH in the range of 3-9; and a reaction time in the range of 1-100 hours. If necessary, immobilized α-amylases can be suitably used for repeated use. Reaction mixtures thus obtained can be subjected to the action of glucoamylase or β-amylase to form and accumulate neotrehalose and α-glycosyl neotrehalose.

The resultant solutions containing neotrehalose, which had been formed by the above-mentioned enzymatic reaction, usually contain about 5-15% neotrehalose, d.s.b. The solutions can be suitably used in the form of liquid, syrup and solid after filtration, purification, concentration and drying. In order to improve the inherent properties of neotrehalose, the solutions are generally separated and purified into a high neotrehalose content product prior to use. Examples of such filtration and purification advantageously usable in the invention are fermentation using yeasts, membrane filtration, separation and sedimentation, crystallization and column chromatography, whereby concomitant saccharides are separated and removed. More particularly, as disclosed in Japanese Patent Laid-Open Nos.23,799/83 and 72,598/88, column chromatography wherein a strongly-acidic cation exchange resin is used can be advantageously used to remove concomitant saccharides in a neotrehalose solution and to obtain a neotrehalose-rich fraction. In this case, fixed-bed method, moving-bed method and semi-moving-bed method can be appropriately used. The high neotrehalose content solution thus obtained can be advantageously concentrated into a high-concentration solution of neotrehalose which can be used to prepare a crystalline neotrehalose by the method as disclosed in Japanese Patent Application No.307,054/90.

The neotrehalose according to the present invention is a disaccharide having a non-reducing activity and satisfiable stability, and a high quality- and rich-sweetness, and these render it readily handleable. As disclosed in Japanese Patent Application No.268,683/91 applied by the present inventors, neotrehalose is orally or parenterally administered to a recipient and well metabolized and advantageously utilized by the recipient as an energy supplement without fear of causing toxicity and side effects. Since neotrehalose is not readily fermented by dental-carries-inducing microorganims, it can be used as a sweetener which causes substantially no dental carries. Furthermore, neotrehalose is a sweetener having a satisfiable stability, and this renders it advantageously useful as a sugar-coating agent for tablets in combination with a binder such as pullulan and hydroxyethyl starch. In addition, neotrehalose has a chemical stability, as well as an osmosis-pressure-regulating ability, filler-imparting ability, gloss-imparting ability, moisture-retaining ability, viscosity-imparting ability, substantial non-fermentability, and crystallization-preventing ability for other saccharides.

Because of the properties of neotrehalose, it can be advantageously used as a sweetener, taste-improving agent, quality-improving agent and stabilizer in compositions such as food products, tobaccos, cigarettes, feeds, pet foods, cosmetics and pharmaceuticals.

The compositions according to the present invention are those which contain an appropriate amount of neotrehalose to exert the inherent activities of neotrehalose. In general, 5% or higher, preferably 10% or higher of neotrehalose, d.s.b, is suitably incorporated into a product to exert a satisfiable effect.

The present compositions can be those prepared with only neotrehalose, and, usually those prepared with neotrehalose in combination with other one or more substances, for example, nutritional substances such as proteins, amino acids, lipids, vitamins and minerals; and substances such as antiseptics, enzymes, hormones and cytokines, to meet to their final use. If necessary, in the preparation of such compositions one or more appropriate substances such as taste-imparting agent, coloring agent, flavor-imparting agent, stabilizer, filler and vehicle can be further added to the compositions before completion of their processings. The resultant compositions can be advantageously formed to meet to their final use. The compositions thus obtained can be orally or parenterally used.

The methods to incorporate neotrehalose in a product usable in the invention are those which can incorporate neotrehalose in it before completion of its processing. For example, conventional methods such as mixing, kneading, dissolving, melting, soaking, spreading, applying, coating, spraying, injecting, crystallizing and solidifying are appropriately chosen.

The following Experiments will explain the present preparation of neotrehalose by using α-amylase.

### Experiment 1

### Screening of neotrehalose-forming enzyme

A saccharogenic α-amylase, an α-amylase (EC 3.2.1.1) specimen commercialized by Ueda Chemical Co. Ltd., Osaka, Japan; "#1500", a β-amylase (EC 3.2.1.2) specimen commercialized by Nagase Biochemicals, Ltd., Kyoto, Japan; "GLUCOZYME", a glucoamylase (EC 3.2.1.3) specimen commercialized by Nagase Biochemicals, Ltd., Kyoto, Japan; an α-glucosidase (EC 3.2.1.20) specimen derived from rice commercialized by Seikagaku-Kogyo Co., Ltd., Tokyo, Japan; and an isoamylase (EC 3.2.1.68) specimen commercialized by Hayashibara Biochemical Laboratories Inc., Okayama, Japan, were used in this experiment. One thousand units per g maltose, d.s.b., of each enzyme was added to a 33% aqueous solution (pH 6.0) containing "MALTOSE HHH" a maltose product commercialized by Hayashibara Biochemical Laboratories Inc., Okayama, Japan, and subjected to an enzymatic reaction at 55°C for 24 hours. The enzymatic reaction was ceased by heating at 100°C for 10 minutes. Each reaction mixture was diluted with 20mM acetate buffer (pH 4.5) into a solution having a concentration of about 5%, d.s.b., added with 20 units/g maltose, d.s.b., of glucoamylase, and subjected to an enzymatic reaction at 40°C for 24 hours. The presence of neotrehalose in each reaction mixture was determined by HPLC wherein "PA-03", a column of YMC Co., Ltd, Kyoto, Japan, and a 70 v/v % aqueous acetonitrile solution as an eluate were used. The results were as shown in Table 1.

**Table 1**

| Enzyme | Neotrehalose content |
|---|---|
| α-Amylase (EC 3.2.1.1) | + + |
| β-Amylase (EC 3.2.1.2) | - |
| Glucoamylase (EC 3.2.1.3) | + |
| α-Glucosidase (EC 3.2.1.20) | + |
| Isoamylase (EC 3.2.1.68) | - |
| The symbol "-" means that neotrehalose was not formed. | |
| The symbol "+" means that the amount of formed neotrehalose was less than 5% against the material, d.s.b. | |
| The symbol "++" means that the amount of formed neotrehalose was 5% or higher against the material, d.s.b. | |

As shown in Table 1, glucoamylase, α-glucosidase and α-amylase formed neotrehalose. As found in a previous publication, glucoamylase and α-glucosidase slightly formed neotrehalose. α-Amylase formed a large amount of neotrehalose. This finding has not yet been known and it is a novel discovery.

Based on this, the present inventors studied α-amylases which form a satisfiable amount of neotrehalose.

### Experiment 2

### Screening of neotrehalose-forming α-amylase

A saccharogenic α-amylase specimen commercialized by Ueda Chemical Co. Ltd., Osaka, Japan; maltotetraose-forming amylase commercialized by Hayashibara Co., Ltd., Okayama, Japan; "SUMIZYME L", a Taka amylase specimen commercialized by Shin Nihon Chemical Ind., Tokyo, Japan; and "NEO-SPITASE" and "SPITASE HS", both of which are thermostable liquefying α-amylase specimens commercialized by Nagase Biochemicals Ltd., Kyoto, Japan, were used in this experiment as an α-amylase. Similarly as in Experiment 1, the presence of neotrehalose was examined, and the results were as shown in Table 2.

**Table 2**

| α-Amylase | Neotrehalose content |
|---|---|
| Saccharogenic α-Amylase | ++ |
| Maltotetraose-forming enzyme | ± |
| Taka amylase | + |
| Thermostable liquefying α-amylase (NEO-SPITASE) | ± |
| Thermostable liquefying α-amylase (SPITASE HS) | ± |
| The symbol "-" means that neotrehalose was not formed. | |
| The symbol "±" means that the amount of formed neotrehalose was trace. | |
| The symbol "+" means that the amount of formed neotrehalose was 5% or lower against the material, d.s.b. | |
| The symbol "++" means that the amount of formed neotrehalose was 5% or higher against the material, d.s.b. | |

As shown in Table 2, all the α-amylase formed neotrehalose in a variety of amounts. Among these α-amylases, the saccharogenic α-amylase formed a relatively-large amount of neotrehalose.

### Experiment 3

### Preparation of neotrehalose by using α-amylase

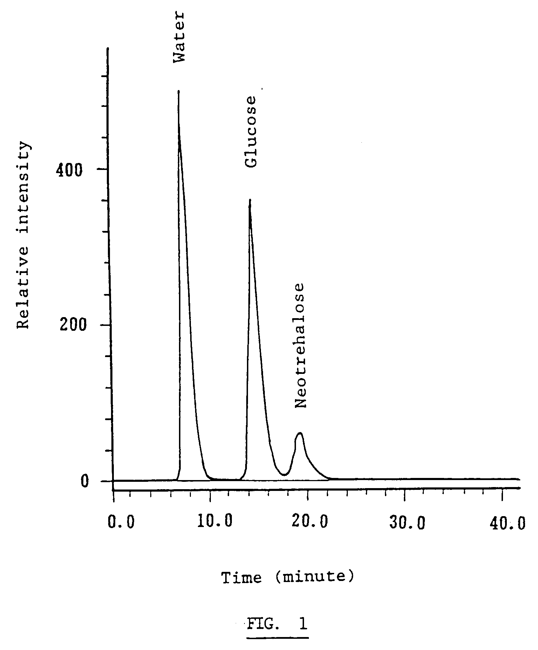

Fifty parts by weight of "MALTOSE H", a maltose product commercialized by Hayashibara Biochemical Laboratories Inc., Okayama, Japan, was dissolved by heating in 50 parts by weight of water, and the solution was heated to 60°C, adjusted to pH 5.2, added with 100 units/g maltose, d.s.b., of a saccharogenic α-amylase specimen derived from Bacillus subtilis, a crystalline enzyme specimen commercialized by Seikagaku-Kogyo Co., Ltd., Tokyo, Japan, and subjected to an enzymatic reaction for 20 hours. Thereafter, the reaction mixture was heated at 100°C for 10 minutes to deactivate the remaining enzyme. The resultant mixture was heated to 55°C, adjusted to pH 4.5, added with 20 units/g of "GLUCOZYME", a glucoamylase specimen commercialized by Nagase Biochemicals, Ltd., Kyoto, Japan, against the solid matter, d.s.b., and subjected to an enzymatic reaction for 16 hours. Thereafter, the reaction mixture was heated at 100°C for 15 minutes to deactivate the remaining enzyme. Similarly as in Experiment 1, the resultant solution was subjected to HPLC, and the chromatogram was as shown in FIG.1 wherein the axis of ordinates indicates a refractive intensity (RI) and the axis of abscissas indicates a time. The solution contained about 12% neotrehalose, d.s.b.

The solution was decolored with an activated charcoal, and desalted and purified with an ion-exchange resin (H⁺- and OH⁻-form). The resultant solution was concentrated to give a concentration of about 40%, d.s.b., and subjected to column chromatography wherein an ion-exchange resin is used to recover a neotrehalose-rich fraction. "Amberlite XT-1016 (Na⁺-form)", a strongly-acidic cation exchange resin commercialized by Japan Organo, Co., Ltd., Tokyo, Japan, was used as a resin for fractionation in a manner that the resin was suspended in water and packed in a jacketted stainless-steel column having an inner diameter of 5.4cm. Four columns of which having a gel-bed depth of 5m were cascaded in series to give a total gel-bed depth of about 20m. The inner-column temperature was kept at 55°C, added with 5 v/v % of a material saccharide solution having a concentration of about 40%, d.s.b., and fed with 55°C water at a flow rate of space velocity (SV) 0.3 to effect fractionation, followed by recovering a neotrehalose-rich fraction. A portion of the fraction containing about 85% neotrehalose, d.s.b., was concentrated into a solution having a concentration of about 75%, d.s.b., which was then allowed to stand at 20°C overnight to form crystal. The resultant crystal was added as a seed crystal to an about 72% solution, d.s.b., which had been prepared from the neotrehalose-rich fraction, and the resultant mixture was gently stirred to effect crystallization. The resultant massecuite was separated to obtain a crystal which was then washed by spraying it with a small amount of water to obtain a high-purity crystal. The resultant crystal was dissolved in water, and, similarly as above recrystallized and separated to obtain an extremely high-purity crystalline neotrehalose with a purity of 99.9%, d.s.b.

### Experiment 4

### Physicochemical properties of neotrehalose

The physicochemical properties of neotrehalose were determined with a high-purity crystalline neotrehalose prepared by the method in Experiment 3. As a result, the physicochemical properties were the same as those shown in Japanese Patent Application No.307,054/90 applied by the present inventors.

The following Examples A and B will explain the present preparation of neotrehalose, and compositions containing said neotrehalose.

### Example A-1

Five parts by weight of "PINE-DEX #4", a dextrin product (a dextrose equivalent (DE) of 18) commercialized by Matsutani Chemical Ind., Co., Ltd., Kyoto, Japan, was dissolved by heating in 5 parts by weight of water, and the solution was adjusted to pH 5.6, heated to 60°C, added with 100 units/g dextrin, d.s.b., of a saccharogenic α-amylase specimen commercialized by Ueda Chemical Co., Ltd., Osaka, Japan, and subjected to an enzymatic reaction for 20 hours. Thereafter, the resultant mixture was heated to deactivated the remaining enzyme. The reaction mixture was in an usual manner decolored with an activated charcoal, and desalted and purified with an ion-exchange resin (H⁺- and OH⁻-form ). The resultant solution was concentrated to obtain a syrup having a concentration of about 75%, d.s.b., in the yield of about 93%. The product contains about 10% neotrehalose, d.s.b., as well as having a rich sweetness, appropriate viscosity and moisture-retaining ability, and because of these the product can be advantageously used in a variety of compositions.

### Example A-2

### Example A-2-(1)

### Preparation of enzyme

A seed culture of a microorganism of the species Bacillus subtilis IFO 14140 (Fukumoto D11) was inoculated to a liquid culture medium as shown in below, and incubated at 27°C for 3 days. The broth was separated by centrifugation, and the resultant supernatant was added with ammonium sulfate, allowed to stand overnight in a cooling room, and subjected to centrifugation, followed by recovering the sediment. The resultant sediment was added with a small amount of 0.1M phosphate buffer (pH 7.0) to dissolve the formed enzyme. The resultant solution was used as an enzyme specimen with an enzyme activity of 340 U/ml.

| Liquid culture medium (pH 7.0) | |
|---|---|
| Solubilized starch | 3.0% |
| Diammonium hydrogenphosphate | 1.0% |
| Corn steep liquor (CSL) | 1.0% |
| Yeast extract | 0.1% |
| Polypeptone | 0.1% |

Throughout the specification, an enzyme activity, i.e. a saccharification power, is determined by adding 0.2ml of an enzyme solution to 5ml solution of one % solubilized starch in 20mM acetate buffer (pH 5.5), subjecting the resultant mixture to an enzymatic reaction at 40°C for 10 minutes, and measuring the amount of the formed reducing sugars by the Somogyi-Nelson method. One unit of the enzyme activity is defined as the amount of enzyme which releases one µmol glucose per minute at 40°C.

### Example A-2

### Example A-2-(2)

### Enzymatic reaction

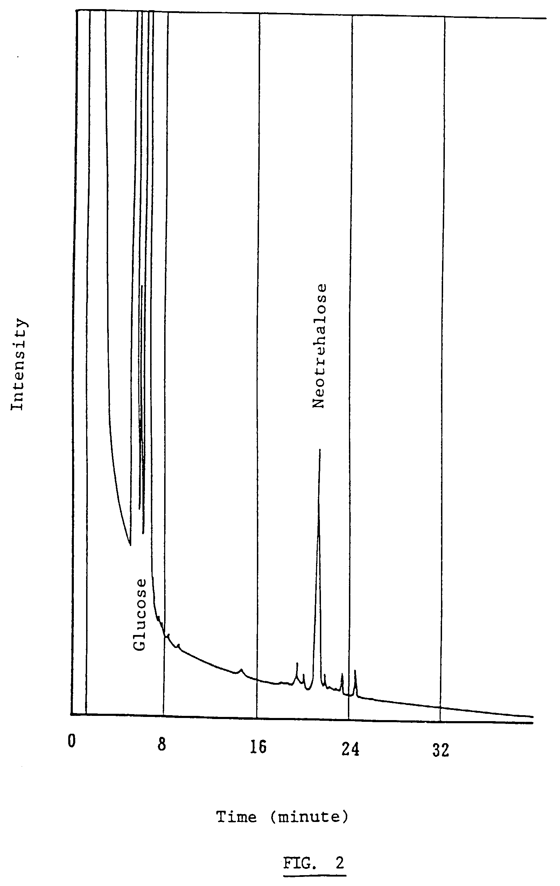

Fifty parts by weight of "MALTOSE H", a maltose product commercialized by Hayashibara Biochemical Laboratories Inc., Okayama, Japan, was added with 50 parts by weight of water and dissolved by heating. The resultant solution was added with 50 units/g maltose, d.s.b., of the enzyme specimen prepared in Example A-2-(1), and subjected to an enzymatic reaction at pH 5.5 and 55°C for 24 hours. The vessel containing the resultant mixture was placed in boiling water for 10 minutes to cease the enzymatic reaction. The mixture thus obtained was diluted with water to give a concentration of about 5%, d.s.b., added with 20 units/g maltose, d.s.b., of glucoamylase, and subjected to an enzymatic reaction at pH 4.5 and 40°C for 24 hours. The reaction mixture thus obtained was subjected to gas chromatography analysis. The results were as shown in FIG. 2. In FIG.2, the axis of ordinates indicates an intensity determined with a flame ionization detector (FID) and the axis of abscissas indicates a time. The reaction mixture contained 7.2% neotrehalose, d.s.b. The reaction mixture was in an usual manner decolored with an activated charcoal, desalted and purified with an ion-exchange resin (H⁺- and OH⁻-form), and concentrated into an about 40% solution, d.s.b., which was then according to the method in Experiment 1 subjected to column chromatography to obtain a neotrehalose-rich fraction containing 52% neotrehalose, d.s.b. The fraction was concentrated into a syrup having a concentration of about 75%, d.s.b. The product has a rich sweetness and an appropriate viscosity and moisture-retaining ability, and because of these it can be advantageously used in a variety of compositions.

### Example A-3

Sixty parts by weight of "SUNMALT®", a maltose product commercialized by Hayashibara Co., Ltd., was dissolved by heating in 40 parts by weight of water, and the solution was heated to 60°C, adjusted to pH 5.2, added with 200 units/g maltose, d.s.b., of a saccharogenic α-amylase specimen commercialized by Ueda Chemical Co., Ltd., Osaka, Japan, and subjected to an enzymatic reaction for 36 hours. Thereafter, the reaction mixture was heated to deactivate the remaining enzyme. The resultant mixture was diluted to give a concentration of about 20%, d.s.b., adjusted to pH 5.0, added with 10 units/g maltose, d.s.b., of glucoamylase, and subjected to an enzymatic reaction for 40 hours. Thereafter, the reaction mixture was heated to deactivate the remaining enzyme.

The mixture thus obtained was in an usual manner decolored with an activated charcoal, desalted and purified with an ion-exchange resin (H⁺- and OH⁻-form), and concentrated into a solution having a concentration of about 60%, d.s.b. Similarly as in Experiment 1 except that "CG 6000 (Na⁺-form)", a strongly-acidic cation exchange resin commercialized by Japan Organo, Co., Ltd, Tokyo, Japan, was used as a resin for fractionation, the resultant solution was subjected to column chromatography to obtain a neotrehalose-rich fraction containing about 90% neotrehalose, d.s.b. The fraction was concentrated to give a concentration of about 76%, d.s.b., placed in a crystallizer, and added with an about 2% seed crystal, d.s.b., to obtain a massecuite having about 45% crystal, d.s.b. The massecuite was sprayed from a nozzle equipped on the upper part of a drying tower at a high-pressure of 150kg/cm², and collected on a metal-wire conveyer provided in the lower part of the spraying tower while sending a 85°C hot-air from the upper part of the spraying tower to the lower part of the spraying tower, and the resultant crystalline powder collected on the conveyer was gradually removed from the drying tower while sending to the crystalline powder a 45°C hot-air from under the conveyer. The crystalline powder was injected to an ageing tower and aged for 10 hours while sending thereto a hot air to complete the crystallization and drying. Thus, a crystalline neotrehalose powder containing other saccharides was obtained. The product has a satisfiable handleability without substantially exhibiting hygroscopicity, and these render it advantageously useful in a variety of compositions as a sweetener, stabilizer, taste-improving agent and quality-improving agent.

### Example A-4

A solution containing about 10% neotrehalose, d.s.b., prepared by the method in Example A-1 was diluted to give a concentration of about 10%, d.s.b., adjusted to pH 4.6, added with 30 units/g dextrin, d.s.b., of glucoamylase, and subjected to an enzymatic reaction for 16 hours. The reaction mixture was heated to deactivate the remaining enzyme. The resultant solution was in an usual manner decolored with an activated charcoal, desalted and purified with an ion-exchange resin (H⁺-and OH⁻-form), and concentrated to give a concentration of about 45%, d.s.b. The resultant solution was subjected to column chromatography to obtain a neotrehalose-rich fraction according to the method in Experiment 1 except that "DOWEX 50W-X4", a strongly-acidic cation exchange resin commercialized by Dow Chemical Co., Midland, Michigan, USA, was used as a resin for fractionation. The fraction containing about 85% neotrehalose, d.s.b., was concentrated to give a concentration of about 85%, d.s.b., fed to a crystallizer, allowed to stand at 20°C for 4 days to effect crystallization and solidification. The solid product thus obtained was pulverized with a pulverizer and dried to obtain a neotrehalose powder containing other saccharides. The product has a satisfiable handleability without substantially exhibiting hygroscopicity, and these render it advantageously useful in a variety of compositions as a sweetener, stabilizer, taste-improving agent and quality-improving agent.

### Example B-1

### Sweetener

One part by weight of a crystalline neotrehalose powder prepared by the method in Example A-3 was mixed to homogeneity with 0.05 parts by weight of "αG Sweet", an α-glycosyl stevioside product commercialized by Toyo Sugar Refining Co., Ltd., Tokyo, Japan, and the resultant mixture was fed to a granulator to obtain a sweetener in the form of granule. The product has a high-quality sweeteness and about 2-fold higher sweetening-power of sucrose, and the calorie per sweetening power lowered to half of that of sucrose. The product is suitably used to impart sweetness to low-caloric food products for persons such as those of overweight and diabetes who are restricted to take calories. Dental-carries-inducing microorganisms less form acids and insoluble glucans when assimilate the product, and this renders it useful as a sweetener for food products which prevent dental caries.

### Example B-2

### Hard candy

One hundred parts by weight of a sucrose solution having a concentration of 55%, d.s.b., was mixed while heating with 30 parts by weight of a syrup containing neotrehalose prepared by the method in Example A-2, and the mixture was concentrated by heating up to give a moisture content of lower than 2%, and mixed with one part by weight of citric acid and adequate amounts of a lemon flavor and coloring agent. The resultant mixture was formed into the captioned product in an usual manner. The product, wherein the crystallization of sugar is prevented, is a high-quality hard candy having a satisfiable biting-property and taste quality.

### Example B-3

### Strawberry jam

One hundred and fifty parts by weight of fresh strawberry, 60 parts by weight of sucrose, 20 parts by weight of maltose, 40 parts by weight of a syrup containing neotrehalose prepared by the method in Example A-2, 5 parts by weight of pectin, and one part by weight of citric acid were sufficiently boiled down in a vessel, and bottled to obtain the captioned product. The product is a satisfiable strawberry-jam having a satisfiable flavor and color.

### Example B-4

### Lactic acid beverage

Ten parts by weight of defatted milk was sterilized by heating at 80°C for 20 minutes, cooled to 40°C, and added with 0.3 parts by weight of a starter, and the mixture was allowed to ferment at about 37°C for 10 hours. Thereafter, the resultant mixture was homogenized, added with 4 parts by weight of a crystalline neotrehalose powder prepared by the method in Example A-4, one part by weight of sucrose, and 2 parts by weight of an isomerized sugar syrup, and the mixture thus obtained was sterilized at 70°C, cooled, added with an adequate amount of a flavoring agent, and bottled to obtain the captioned product. The product is a high-quality lactic acid beverage having a well harmonized sourness with flavor and sweetness.

### Example B-5

### Condensed milk with sugar

To 100 parts by weight of milk dissolved 3 parts by weight of a syrup containing neotrehalose, prepared by the method in A-2, and one part by weight of sucrose, and the resultant solution was sterilized by heating with a plate heater, concentrated to give a concentration of about 70%, d.s.b., and aseptically bottled to obtain the captioned product. The product has a mild sweeteness and a satisfiable flavor, and these render it advantageously useful as a seasoning in foods for infants, fruits, coffee, cocoa and tea.

### Example B-6

### Fruit juice powder

Thirty-three parts by weight of a fruit juice powder, prepared by spray-drying a fruit juice, was added with 50 parts by weight of a crystalline neotrehalose powder prepared by the method in Example A-3, 10 parts by weight of sucrose, 0.65 parts by weight of anhydrous citric acid, 0.1 part by weight of malic acid, 0.1 part by weight of L-ascorbic acid, 0.1 part by weight of sodium citrate, 0.5 parts by weight of pullulan, and an adequate amount of a powdered flavor, and the resultant was mixed to homogeneity, pulverized to obtain a pulverulent product which was then fed to a fluidized-bed granulator, sprayed with a high neotrehalose content solution prepared by the method in Example A-3 as a binder, and granulated for 30 minutes while sending thereto a 40°C hot-air at a flow rate of 150m³/min. Thereafter, the resultant powder was weighed and packed to obtain the captioned product. The product is a fruit juice powder containing about 30% fruit juice, d.s.b. The product free of unsatisfiable smell and odor is stable for a relatively-long period of time without exhibiting moisture absorption and solidification.

### Example B-7

### Chocolate

Forty parts by weight of cacao paste, 10 parts by weight of cacao butter, and 50 parts by weight of a crystalline neotrehalose powder prepared by the method in Example A-4 were passed through a refiner to lower the particle size, fed to a conche, and kneaded at 50°C over 2 days. During this procedure, 0.5 parts by weight of lecithin was added to the mixture and sufficiently dispersed by mixing therein. Thereafter, the resultant mixture was heated to 31°C with a thermoregulator, poured in a molder immediately before the solidification of butter, deaerated with a vibrator, and passed through a tunnel kept at 10°C for 20 minutes to effect solidification. The solidified product was removed from the molder and packed to obtain the captioned product. The product has a satisfiable color, gloss, texture, high quality, mild sweeteness, and meltability in the mouth without substantially exhibiting hygroscopicity.

### Example B-8

### Chewing gum

Three parts by weight of a gum base was melted by heating until it was softened, and admixed with 4 parts by weight of sucrose, 3 parts by weight of a crystalline neotrehalose powder prepared by the method in Example A-3, and adequate amounts of a flavoring agent and coloring agent. The resultant mixture was in an usual manner kneaded with a role, formed and packed to obtain the captioned product. The product is a chewing gum having a satisfiable texture and flavor.

### Example B-9

### Cream filling

One hundred parts by weight of corn starch, 100 parts by weight of a syrup containing neotrehalose prepared by the method in Example A-2, 80 parts by weight of maltose, 20 parts by weight of sucrose, and one part by weight of salt were mixed to homogeneity, and the mixture was admixed with 280 parts by weight of egg. The resultant mixture was gradually added with 1,000 parts by weight of a boiling milk and stirred while heating. The heating was stopped when the corn starch was gelatinized to show the whole content semitransparent, cooled, added with an adequate amount of vanilla flavor, weighed, injected, and packed to obtain the captioned product. The product has a smooth gloss, mild sweetness and satisfiable taste.

### Example B-10

### "Uiro-no-moto" (premix of sweet rice jelly)

Ninety parts by weight of rice powder, 20 parts by weight of corn starch, 120 parts by weight of a crystalline powder prepared by the method in Example A-4, and 4 parts by weight of pullulan were mixed to homogeneity to obtain the captioned product. The product was kneaded with adequate amounts of water and a powdered green tea, and the resultant mixture was placed in a vessel and steamed for 60 minutes to obtain a sweet rice jelly with the powdered green tea. The product has a satisfiable gloss, biting property and flavor. The retrogradation of starch in the product is well prevented to exert a relatively-long shelf-life.

### Example B-11

### Solid preparation for intubation feeding

A composition consisting of 500 parts by weight of a crystalline neotrehalose prepared by the method in Experiment 3, 270 parts by weight of powdered egg, 209 parts by weight of defatted milk powder, 4.4 parts by weight of sodium chloride, 1.8 parts by weight of potassium chloride, 4 parts by weight of magnesium chloride, 0.01 part by weight of thiamine, 0.1 part by weight of ascorbic acid, 0.6 parts by weight of vitamin E acetate, and 0.04 parts by weight of nicotinamide was prepared, and 25g aliquots of the composition were injected to small moistureproof laminated bags, and heat sealed to obtain the captioned product. One bag of the product is dissolved in about 150-300ml water into a food for intubation feeding which is orally administered to a recipient or administered to nasal cavity, stomach and intestines of a recipient by intubation feeding. The product can be advantageously used as a composition for supplementing energy.

### Example B-12

### Transfusion

A crystalline neotrehalose prepared by the method in Experiment 3 was dissolved in water to give a concentration of 10 w/v %, and the resultant solution was in an usual manner membrane filtered, aseptically injected to plastic bottles, and cap sealed to obtain the captioned product. The product is a transfusion having a satisfiable stability, and suitably intravenously and intraperitoneally administered to a recipient. A 10 w/v % solution of the product is isotonic to the blood so that the product can supplement energy to a recipient at 2-fold higher concentration than that of glucose.

### Example B-13

### Transfusion

A crystalline neotrehalose prepared by the method in Experiment 3 and a composition consisting of amino acids as shown below are respectively dissolved by mixing in water into 5 w/v % and 3 w/v % solutions. Thereafter, the solutions were purified similarly as in Example B-12, injected to plastic bags, and sealed to obtained the captioned product.

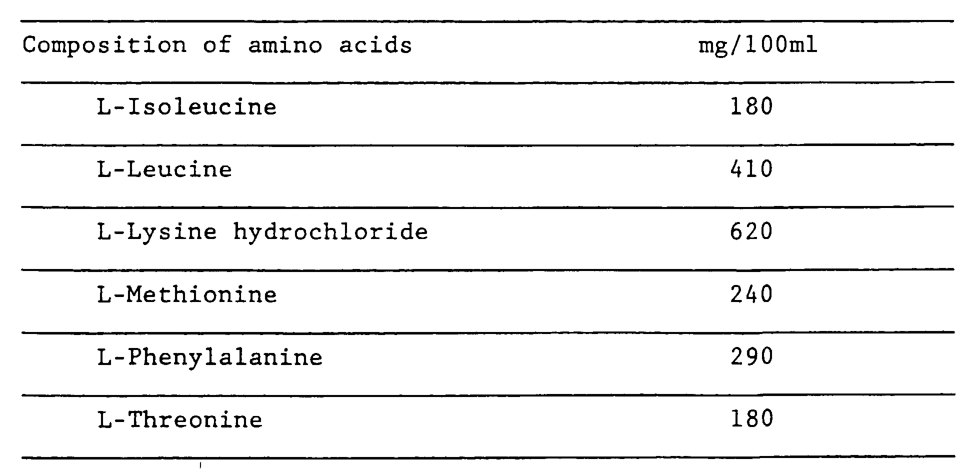

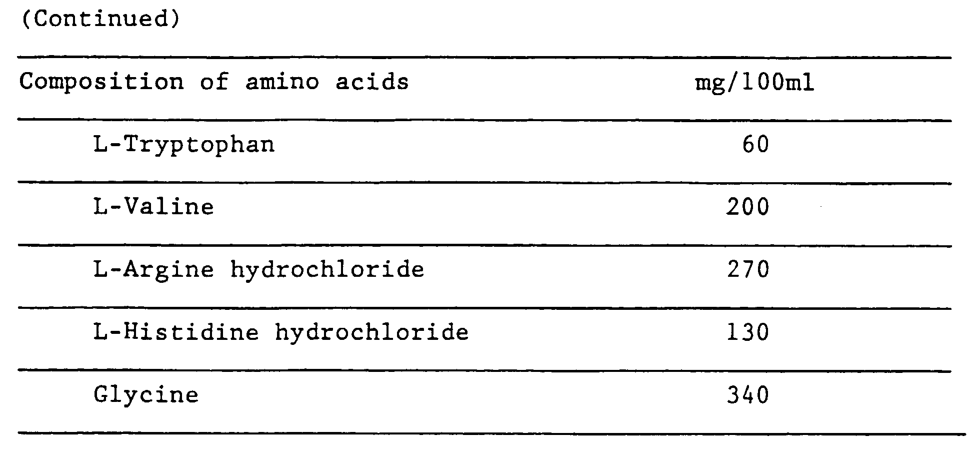

The product, a complex transfusion of a saccharide and amino acids, is a stable transfusion which is suitably intravenously and intraperitoneally administered to a recipient because the neotrehalose contained in the product shows no reducing activity. The product can be advantageously used as an energy-supplementing composition which supplements energy and amino acids to a recipient.

### Example B-14

### Ointment for traumatic injury

Five hundred parts by weight of a crystalline powder prepared by the method in Example A-4 was admixed with 50 parts by weight of methanol in which 3 parts by weight of iodine had been dissolved, and the resultant mixture was admixed with 200 parts by weight of a 10% aqueous pullulan solution to obtain the captioned product having an appropriate extensibility and adhesiveness. The product reduces the healing time and completely heals an affected part because the iodine exerts a bactericidal action and the neotrehalose acts as a composition which supplements energy to living cells.

### Example B-15

### Sugar-coated tablet

A 150mg crude tablet as a center was sugar coated until it reached to about 230mg with a first solution consisting of 40 parts by weight of a crystalline neotrehalose prepared by the method in Experiment 3, 2 parts by weight of pullulan having an average molecular weight of 200,000, 30 parts by weight of water, 25 parts by weight of talc, and 3 parts by weight of titanium oxide. The tablet thus obtained was coated with a second solution consisting of 65 parts by weight of the same crystalline neotrehalose as above, one part by weight of the same pullulan as above, and 34 parts by weight of water, and further coated with a wax to obtain the captioned product having a satisfiable gloss and appearance. The sugar-coating steps of the product is readily feasible, and the product has a satisfiable shock-tolerance and retains a high quality for a relatively-long period of time. Thus, the product can be advantageously used as an energy-supplementing composition.

As obvious above, the present neotrehalose is prepared in a relatively-high yield by allowing α-amylase to act on an amylaceous substance to form neotrehalose, and the separation and purification of said neotrehalose in the resultant reaction mixture is readily feasible. The neotrehalose thus obtained is a non-reducing disaccharide which has an excellent stability and a high quality- and rich-sweeteness. The neotrehalose is orally and parenterally used, and well metabolized and advantageously utilized by a recipient as an energy supplement without fear of causing toxicity and side effects. Neotrehalose in the form of crystal readily dissolves in water but it is substantially non-hygroscopic so that it is readily handleable. Neotrehalose has a satisfiable chemical stability, as well as other properties such as a osmosis-regulating activity, filler-imparting ability, gloss-imparting ability, moisture-retaining ability, viscosity-imparting ability, crystallization-preventing ability for other saccharides, and substantial non-fermentability. These properties render neotrehalose advantageously useful in preparations of a variety of compositions as a sweetener, taste-improving agent, quality-improving agent and stabilizer.

Accordingly, the establishment of the preparation of neotrehalose and its uses according to the present invention has a great significance in the fields of food products, cosmetics and pharmaceuticals.

## Claims

1. A process for preparing neotrehalose, which comprises:
(a) allowing α-amylase to act on an amylaceous substance in a solution to form neotrehalose; and
(b) recovering said neotrehalose.

2. A process according to claim 1, wherein said α-amylase is a saccharogenic α-amylase.

3. A process according to claim 1 or claim 2, wherein the concentration of said amylaceous substance is in the range of 1-80 w/w %, based on the weight of the dry solid.

4. A process according to any one of claims 1 to 3, wherein the pH in the step (a) is in the range of 3-9.

5. A process according to any one of the preceding claims, wherein the temperature in the step (a) is in the range of 20-80°C.

6. A process according to any one of the preceding claims, wherein the enzymatic reaction-time in the step (a) is in the range of 1-100 hours.

7. A process according to any one of the preceding claims, wherein said α-amylase is immobilized.

8. A process according to any one of the preceding claims, wherein the resultant solution in the step (a) contains about 5-15 w/w % neotrehalose, based on the weight of the dry solid.

9. A process according to any one of the preceding claims, wherein the step (b) contains steps of subjecting the resultant solution containing neotrehalose in the step (a) to a separation and purification, recovering the resultant neotrehalose-rich fraction, and concentrating the fraction to obtain a neotrehalose concentrate.

10. A process according to claim 9, wherein the neotrehalose concentrate comprises a high neotrehalose content syrup.

11. A process according to claim 9, which further comprises:
crystallizing the neotrehalose in the resultant concentrate and
recovering the resultant crystalline neotrehalose.

12. A process according to any one of claims 9 to 11, wherein said separation and purification is a member selected from the group consisting of fermentation using yeasts, membrane filtration, separation and sedimentation, crystallization, and column chromatography wherein a strongly-acidic cation exchange resin is used.

13. A process for preparing a composition comprising neotrehalose, which process comprises:
(i) preparing neotrehalose in accordance with the process of any of the preceding claims; and
(ii) preparing a composition comprising the neotrehalose.

14. A process according to claim 13, wherein the composition comprising the neotrehalose contains at least 5 w/w % neotrehalose, based on the weight of the dry solid.

## Patentansprüche

1. Verfahren zum Herstellen von Neothrehalose, das umfaßt:
a) Einwirkenlassen von α-Amylase auf eine stärkehaltige Substanz in einer Lösung zur Bildung von Neothrehalose; und
b) Wiedergewinnen der Neothrehalose.

2. Verfahren nach Anspruch 1, bei welchem die α-Amylase eine Saccharogen-α-Amylase ist.

3. Verfahren nach Anspruch 1 oder 2, bei welchem die Konzentration der stärkehaltigen Substanz im Bereich von 1 - 80 Gew.-%, bezogen auf das Gewicht des trockenen Feststoffs, ist.

4. Verfahren nach einem der Ansprüche 1 bis 3, bei welchem das pH im Schritt (a) im Bereich von 3 - 9 liegt.

5. Verfahren nach einem der vorangehenden Ansprüche, bei welchem die Temperatur im Schritt (a) im Bereich von 20 - 80°C liegt.

6. Verfahren nach einem der vorangehenden Ansprüche, bei welchem die enzymatische Reaktionszeit im Schritt (a) im Bereich von 1 - 100 Stunden liegt.

7. Verfahren nach einem der vorangehenden Ansprüche, bei welchem die α-Amylase immobilisiert wird.

8. Verfahren nach einem der vorangehenden Ansprüche, bei welchem die erhaltene Lösung im Schritt (a) etwa 5 - 15 Gew.-% Neothrehalose enthält, bezogen auf das Gewicht des trockenen Feststoffs.

9. Verfahren nach einem der vorangehenden Ansprüche, bei welchem der Schritt (b) die Schritte umfaßt: Unterwerfen der Neothrehalose enthaltenden, sich im Schritt (a) ergebenden Lösung einer Trennung und Reinigung, Wiedergewinnen der erhaltenen neothrehalosereichen Fraktion und Konzentrieren der Fraktion zur Erzielung eines Neothrehalosekonzentrats.

10. Verfahren nach Anspruch 9, bei welchem das Neothrehalosekonzentrat einen Sirup mit hohem Neothrehalosegehalt enthält.

11. Verfahren nach Anspruch 9, welches ferner umfaßt:
Kristallisieren der Neothrehalose im erhaltenen Konzentrat und
Wiedergewinnen der erhaltenen kristallinen Neothrehalose.

12. Verfahren nach einem der Ansprüche 9 bis 11, bei welchem die Trennung und Reinigung in einer Art erfolgt, die aus der Gruppe gewählt ist, welche besteht aus Fermentation unter Verwendung von Hefen, Membranfiltration, Trennung und Sedimentation, Kristallisation und Säulenchromatographie, wobei ein stark saures Kationenaustauscherharz verwendet wird.

13. Verfahren zum Herstellen einer Neothrehalose enthaltenden Zusammensetzung, welches umfaßt:
(i) Herstellen von Neothrehalose nach dem Verfahren gemäß einem der vorangehenden Ansprüche; und
(ii) Herstellen einer die Neothrehalose enthaltenden Zusammensetzung.

14. Verfahren nach Anspruch 13, bei welchem die die Neothrehalose enthaltende Zusammensetzung wenigstens 5 Gew.-% Neothrehalose enthält, bezogen auf das Gewicht des trockenen Feststoffs.

## Revendications

1. Procédé de préparation de néotréhalose, comprenant les étapes consistant à :
(a) laisser agir une α-amylase sur une substance amylacée en solution pour former du néotréhalose ; et
(b) récupérer ledit néotréhalose.

2. Procédé selon la revendication 1, dans lequel ladite α-amylase est une α-amylase saccharogène.

3. Procédé selon la revendication 1 ou la revendication 2, dans lequel la concentration de ladite substance amylacée est comprise entre 1 et 80 % en poids/poids sur la base du poids du solide sec.

4. Procédé selon l'une quelconque des revendications 1 à 3, dans lequel le pH pendant l'étape (a) est compris entre 3 et 9.

5. Procédé selon l'une quelconque des revendications précédentes, dans lequel la température pendant l'étape (a) est comprise entre 20 et 80°C.

6. Procédé selon l'une quelconque des revendications précédentes, dans lequel le temps de réaction enzymatique pendant l'étape (a) est compris entre 1 et 100 heures.

7. Procédé selon l'une quelconque des revendications précédentes, dans lequel ladite α-amylase est immobilisée.

8. Procédé selon l'une quelconque des revendications précédentes, dans lequel la solution résultante lors de l'étape (a) contient environ 5 à 15 % en poids/poids de néotréhalose sur la base du poids du solide sec.

9. Procédé selon l'une quelconque des revendications précédentes, dans lequel l'étape (b) comprend les étapes consistant à soumettre la solution résultante contenant du néotréhalose, obtenue pendant l'étape (a), à une séparation et une purification, récupérer la fraction résultante riche en néotréhalose et concentrer cette fraction pour obtenir un concentré de néotréhalose.

10. Procédé selon la revendication 9, dans lequel le concentré de néotréhalose est constitué par un sirop à forte teneur en néotréhalose.

11. Procédé selon la revendication 9, comprenant, en outre :
la cristallisation du néotréhalose dans le concentré résultant et
la récupération du néotréhalose cristallin résultant.

12. Procédé selon l'une quelconque des revendications 9 à 11, dans lequel lesdites opérations de séparation et de purification sont des éléments choisis dans l'ensemble constitué par la fermentation utilisant des levures, la filtration par membrane, la séparation et la sédimentation, la cristallisation et la chromatographie sur colonne utilisant une résine fortement acide pour l'échange de cations.

13. Procédé de préparation d'une composition comprenant du néotréhalose, comprenant :
(i) la préparation de néotréhalose conformément au procédé selon l'une quelconque des revendications précédentes, et
(ii) la préparation d'une composition comprenant le néotréhalose.

14. Procédé selon la revendication 13, dans lequel la composition comprenant le néotréhalose contient au moins 5 % en poids/poids de néotréhalose, sur la base du poids du solide sec.
